(19) Europäisches Patentamt
European Patent Office
Office européen des brevets

(11) **EP 4 641 170 A1**

(12) **EUROPEAN PATENT APPLICATION**
published in accordance with Art. 153(4) EPC

(43) Date of publication:
**29.10.2025 Bulletin 2025/44**

(21) Application number: **23906557.6**

(22) Date of filing: **17.11.2023**

(51) International Patent Classification (IPC):
**G01N 17/00** (2006.01)   **G01N 3/20** (2006.01)

(52) Cooperative Patent Classification (CPC):
**G01N 3/20; G01N 17/00**

(86) International application number:
**PCT/JP2023/041468**

(87) International publication number:
**WO 2024/135188 (27.06.2024 Gazette 2024/26)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **20.12.2022 JP 2022203301**

(71) Applicant: **NIPPON STEEL CORPORATION**
**Chiyoda-ku**
**Tokyo 100-8071 (JP)**

(72) Inventors:
• **TABATA, Shinichiro**
**Tokyo 100-8071 (JP)**
• **YOSHIKAWA, Nobuo**
**Tokyo 100-8071 (JP)**
• **KUSUMI, Kazuhisa**
**Tokyo 100-8071 (JP)**
• **IRIKAWA, Hideaki**
**Tokyo 100-8071 (JP)**
• **TAKABAYASHI, Kensuke**
**Tokyo 100-8071 (JP)**

(74) Representative: **Vossius & Partner**
**Patentanwälte Rechtsanwälte mbB**
**Siebertstrasse 3**
**81675 München (DE)**

(54) **HYDROGEN EMBRITTLEMENT EVALUATION METHOD AND EVALUATION DEVICE**

(57) Provided are a method for evaluation of hydrogen embrittlement, comprising applying stress to a test piece of a steel material so as to obtain a value of stress triaxiality selected in advance from a range of 0.30 to 0.80, and determining a critical hydrogen content of the steel material, and a device for evaluation of hydrogen embrittlement, comprising a holder for holding a test piece of a steel material, a stress applying means for applying stress to the test piece, a display part for displaying an amount of displacement of the test piece to which stress has been applied, and a transmitting means for mechanically transmitting the amount of displacement of the test piece to the display part, wherein the device is able to control the stress applied by the stress applying means so that the amount of displacement of the test piece becomes an amount of displacement corresponding to a predetermined value of stress triaxiality.

Fig. 3

EP 4 641 170 A1

## Description

FIELD

The present invention relates to a method for evaluation and device for evaluation of hydrogen embrittlement.

BACKGROUND

In recent years, in the automobile industry, lighter weight of car bodies is being sought from the viewpoint of improving fuel economy. To achieve both lighter weight of car bodies and collision safety, making the steel sheet used higher in strength is one effective method. With this as a background, high strength steel materials are being developed. In high strength steel materials, hydrogen embrittlement cracking (also referred to as "delayed fracture", etc.) sometimes becomes a problem. "Hydrogen embrittlement cracking" is the phenomenon of a steel member to which high stress acts in situations of use, etc., suddenly fracturing due to hydrogen penetrating the steel from the environment.

In general, it is known that hydrogen embrittlement cracking more easily occurs the higher the strength of a steel material. Therefore, in development of a high strength steel material, more accurately evaluating hydrogen embrittlement is extremely important in developing and selecting suitable materials.

In relation to this, PTL 1 describes a method for evaluation of a delayed fracture property of high strength steel sheet comprising bending high strength steel sheet into a V-shape to prepare a test piece, changing an opening angle of a bent part of the test piece by exactly a predetermined angle to constrain the test piece in a state in which residual stress is imparted, setting the test piece in that constrained state in a hydrogen penetrating environment, evaluating the delayed fracture property of the high strength steel sheet by the state of formation of cracks of the test piece, finding the relationship between a maximum residual stress occurring at surface most layers of the sheet front and back at the bent part of the test piece corresponding to the amount of change of the opening angle and that amount of change, bending the high strength steel sheet to a target product shape based on the found relationship, finding a range of the amount of change of the opening angle at which the maximum residual stress is present within the range of residual stress occurring in the bent part of the product shape, setting the predetermined angle so that it becomes within the range of the amount of change of the opening angle found, then performing the evaluation. Further, PTL 1 teaches that according to the above method, the amount of change of the opening angle with respect to the test piece bent in a V-shape is set to a suitable value at all times, whereby it becomes possible to improve the accuracy of evaluation of the delayed fracture property at the bent part of the high strength steel sheet.

PTL 2 describes a method for evaluation of a delayed fracture property for evaluating a delayed fracture property of a metal sheet, the method for evaluation of a delayed fracture property of a metal sheet comprising fabricating a test piece by performing a first bending step of performing a bending operation for bending the metal sheet in one direction of the sheet thickness direction, then performing one time or more a second bending step of performing a bending operation for bending in an opposite direction of the sheet thickness direction from one bending operation before and evaluating the delayed fracture property by the fabricated test piece. Further, PTL 2 teaches that according to the above method, the delayed fracture can be evaluated under conditions where strain and residual stress are introduced by bending and unbending the metal sheet and, as a result, a delayed fracture property of the metal sheet can be evaluated in an environment closer to an actual part and application of high strength steel sheet to a car body can be made easier.

PTL 3 describes a method for surface treatment of a test piece for evaluation of hydrogen embrittlement resistance comprising electroplating a test piece comprised of a metal material impregnated with hydrogen in a plating bath containing $ZnCl_2$ in more than 80 g/l to 300 g/l or less, a gloss agent in 10 to 50 ml/l, and one or both of $NH_4Cl$ and $KCl$ in a total of 100 to 300 g/l. Further, PTL 3 teaches that, according to the above method for surface treatment, it becomes possible to provide a method for evaluation of hydrogen embrittlement resistance enabling measurement by a high accuracy and high efficiency of a critical amount of diffusible hydrogen, a constant load test, or other delayed fracture test using a test piece treated by galvanization, which is safer and lower in environmental load than a method using cadmium plating, and higher in ability to prevent release of hydrogen during the test than in the past, and that the contribution to industry is extremely remarkable.

[CITATIONS LIST]

[PATENT LITERATURE]

[PTL 1] Japanese Unexamined Patent Publication No. 2017-142086
[PTL 2] Japanese Unexamined Patent Publication No. 2022-024814

# EP 4 641 170 A1

[PTL 3] Japanese Unexamined Patent Publication No. 2008-261742

SUMMARY

[TECHNICAL PROBLEM]

Hydrogen embrittlement cracking occurs more easily the higher the strength of a steel material. On the other hand, in the automobile industry, etc., further lighter weight of steel materials is being sought. To achieve such lighter weight, steel materials have to be made even higher in strength than up to now. In relation to this, in automobiles, many parts made from steel materials and differing in shapes and properties are being used. There can be various stress states depending on the parts and portions of those parts. Therefore, from the viewpoint of suitable development and selection of high strength steel materials, there is a high need for a method for evaluation of hydrogen embrittlement considering such differing stress states.

Therefore, the present invention has as its object the provision of a method for evaluation and a device for evaluation enabling evaluation of hydrogen embrittlement considering the stress state of a steel material.

[SOLUTION TO PROBLEM]

The inventors engaged in studies for achieving this object and as a result discovered that by applying suitable stress to a steel material while considering stress triaxiality, it is possible to simulate various stress states in actual parts and thereby completed the present invention.

The present invention able to achieve this object is as follows:

(1) A method for evaluation of hydrogen embrittlement, comprising applying stress to a test piece of a steel material so as to obtain a value of stress triaxiality selected in advance from a range of 0.30 to 0.80, and determining a critical hydrogen content of the steel material.
(2) The method according to (1), wherein the stress is applied by bending and the value of the stress triaxiality is controlled by changing a ratio w/t of width "w" and thickness "t" of the test piece.
(3) The method according to (1), wherein the stress is applied by spherical punching.
(4) The method according to any one of (1) to (3), wherein determining the critical hydrogen content of the steel material is performed by using a test piece galvanized subsequent to introduction of hydrogen.
(5) The method according to any one of (1) to (4), further comprising calculating stress triaxiality and maximum main stress at a specific portion in a part to be evaluated for hydrogen embrittlement before applying stress to the test piece of the steel material,
wherein applying stress to the test piece of the steel material comprises applying a stress corresponding to the calculated stress triaxiality and maximum main stress to the test piece of the steel material.
(6) A device for evaluation of hydrogen embrittlement, comprising

a holder for holding a test piece of a steel material,
a stress applying means for applying stress to the test piece,
a display part for displaying an amount of displacement of the test piece to which stress has been applied, and
a transmitting means for mechanically transmitting the amount of displacement of the test piece to the display part,
wherein the device is able to control the stress applied by the stress applying means so that the amount of displacement of the test piece becomes an amount of displacement corresponding to a predetermined value of stress triaxiality.

(7) The device according to (6), wherein the predetermined value of stress triaxiality is a value selected from a range of 0.30 to 0.80.
(8) The device according to (6) or (7), wherein the holder for holding the test piece of the steel material is configured to be able to hold a test piece with a ratio w/t of width "w" and thickness "t" changed within a range of 1.00 to 30.00.

[ADVANTAGEOUS EFFECTS OF INVENTION]

According to the present invention, it is possible to provide a method for evaluation and a device for evaluation enabling evaluation of hydrogen embrittlement considering a stress state of a steel material.

BRIEF DESCRIPTION OF DRAWINGS

FIG. 1 is a schematic view showing a conventional method for evaluation of hydrogen embrittlement.
FIG. 2 is a schematic view showing a conventional device for evaluation of hydrogen embrittlement.
FIG. 3 is a schematic view showing a device for evaluation of hydrogen embrittlement according to an embodiment of the present invention.

DESCRIPTION OF EMBODIMENTS

<Method for Evaluation of Hydrogen Embrittlement>

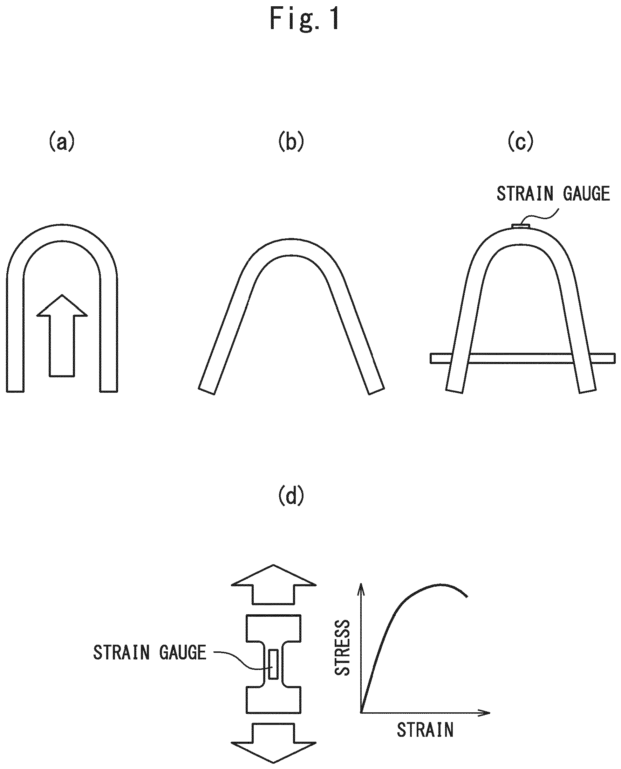

The method for evaluation of hydrogen embrittlement according to an embodiment of the present invention comprises applying stress to a test piece of a steel material so as to obtain a value of stress triaxiality selected in advance from a range of 0.30 to 0.80, and determining a critical hydrogen content of the steel material.
In the past, the methods for evaluation of hydrogen embrittlement generally have been methods using strain gauges, etc. FIG. 1 is a schematic view showing a conventional method for evaluation of hydrogen embrittlement. Explained specifically, in the conventional method for evaluation of hydrogen embrittlement, for example, a short strip shaped test piece having a predetermined length and width is taken from a steel sheet. The test piece is bent 180° by a punch (FIG. 1(a)), then the sprung back U-bent test piece (FIG. 1(b)) is fastened by a bolt and nuts to apply stress (FIG. 1(c)). At this time, a strain gauge is attached to the top part of the U-bent test piece and predetermined stress is applied by control of the amount of strain. The stress applied, as shown in FIG. 1(d), is determined utilizing a stress-strain curve obtained by a tensile test separately performed in advance using the same strain gauge. However, this time, the inventors discovered that in the case of such an evaluation method, even if the values of the stress based on the amount of strain are the same between a U-bending test or other test and a tensile test by uniaxial tension performed separately, sometimes the stress states of the test piece at the different tests will be differ. In relation to this, they discovered that sometimes suitable stress is not necessarily applied in a test for evaluation of hydrogen embrittlement. Furthermore, the inventors discovered that sometimes the stress states will similarly differ between a test piece used in the method for evaluation of hydrogen embrittlement and an actual part. In such a case as well, they discovered that sometimes it is not possible to suitably evaluate a steel material for hydrogen embrittlement.
Therefore, the inventors engaged in studies for realization of more accurate evaluation of the hydrogen embrittlement of a steel material from the viewpoint of applying suitable stress to the steel material while considering the stress state at the actual part. As a result, the inventors discovered that, in place of the conventional method of evaluation using only a strain gauge, it is possible to apply suitable stress to the steel material considering stress triaxiality to thereby enable various stress states at actual parts to be simulated and as a result possible to calculate an upper limit content of hydrogen where hydrogen embrittlement cracking does not occur in a steel material, i.e., a critical hydrogen content, more accurately compared with the conventional evaluation method. Specifically, first, "stress triaxiality" is an indicator expressing the multiaxiality of stress and a parameter obtained by dividing the average stress (of three axes) by the equivalent stress, as represented by the following formula 1:

$$\text{Stress triaxiality} = \text{average stress}/\text{equivalent stress} \quad \cdots \text{ formula 1}$$

Further, the average stress and equivalent stress are represented by the following formulas 2 and 3 using three components $\sigma_1$, $\sigma_2$, and $\sigma_3$ of the main stress:
[Mathematical 1]

$$\text{Average stress} = \frac{1}{3}(\sigma_1 + \sigma_2 + \sigma_3) \quad \cdots \text{ formula 2}$$

[Mathematical 2]

$$\text{Equivalent stress} = \sqrt{\frac{1}{2}\{(\sigma_1 - \sigma_2)^2 + (\sigma_2 - \sigma_3)^2 + (\sigma_3 - \sigma_1)^2\}} \quad \cdots \text{ formula 3}$$

As clear from formulas 1 to 3, for example, in the case of uniaxial tension, $\sigma_1 = 1$, $\sigma_2 = \sigma_3 = 0$, and therefore the stress triaxiality becomes 0.33. On the other hand, for example, if tensile stress occurs in multiple directions, the average stress becomes larger and the equivalent stress becomes smaller since it is the difference of the main stresses. As a result, the stress triaxiality becomes a value higher than 0.33. The stress triaxiality changes in value in accordance with the stress state of the material in this way. The multiaxiality becomes larger compared to the 0.33 of uniaxial tension. Its value becomes higher in the positive direction. For example, with equibiaxial tension, the value becomes 0.66. By applying triaxial tension, a further higher stress triaxiality can be realized. Conversely, in the case of the compressive stress state, this becomes a negative value. For example, in the case of uniaxial compression, the stress triaxiality becomes - 0.33.

Here, hydrogen embrittlement cracking remarkably occurs if mainly a high tensile stress acts on the steel material. Therefore, as hydrogen embrittlement, basically evaluation in a stress state where the stress triaxiality is in a positive range is studied. However, in the case of actual hydrogen embrittlement cracking, for example, in the case of steel sheet for automobiles, various stress states can arise not only at the time of production, but also at the time of corrosion in the usage environment, etc. For example, in the case of high strength cold rolled steel sheet, sometimes hydrogen penetrates the steel from moisture in the atmosphere, etc., in the annealing step at the time of production. In this case, after that, when cold working the obtained high strength cold rolled steel sheet or assembling it into a part by welding, there is a risk of the tensile stress applied in these operations causing hydrogen embrittlement cracking. Further, in the case of a hot stamping material, at the time of hot stamping, the material is heated to an approximately 900°C high temperature, and therefore sometimes hydrogen penetrates the steel from the moisture in the atmosphere at that time, etc. In this case, after that, when assembling the obtained hot stamped body into a part by spot welding, etc., there is similarly a risk of the tensile stress applied due to such an operation causing hydrogen embrittlement cracking. Furthermore, in a step of painting a car body, sometimes hydrogen penetrates the steel at the time of chemical conversion and electrodeposition. Furthermore, in the usage environment after assembly into a car body as well, there is a risk of the hydrogen generated at the time of corrosion penetrating the steel and the stress applied at the time of use, etc., causing hydrogen embrittlement cracking. As explained above, there are various scenarios in which hydrogen embrittlement cracking occurs depending on the steel material even just at the time of production and the stress states at that time. Furthermore, the stress state can greatly change depending on the part to which the steel material is applied or the portion of the same. In addition, in the case of hydrogen embrittlement cracking caused by corrosion in the usage environment, the stress state changes by the pits formed due to corrosion, and therefore naturally a need arises to consider stress states different from the time of production.

Therefore, the inventors discovered that by applying stress to a test piece of a steel material so as to obtain a value of stress triaxiality selected in advance from a range of 0.30 to 0.80, it is possible to cover various stress states at actual parts and that by determining the critical hydrogen content of a steel material in such stress states, it is possible to realize more suitable evaluation of hydrogen embrittlement of a steel material. In the past, hydrogen embrittlement in a steel material is usually evaluated considering only the absolute value of stress applied to a steel material. Therefore, the fact that by applying suitable stress considering the stress state of a steel material and applying such stress by utilizing stress triaxiality to thereby determine the critical hydrogen content of the steel material, it is possible to realize suitable evaluation of the hydrogen embrittlement of a steel material was first made clear by the inventors this time. Further, according to the method for evaluation of hydrogen embrittlement according to an embodiment of the present invention, it is also possible to apply stress corresponding to a stress state not covered by a conventional evaluation method. Therefore, it is possible to suitably and reliably create in a test piece of a steel material a stress state in which hydrogen embrittlement cracking can occur in an actual part. For this reason, the method for evaluation of hydrogen embrittlement according to an embodiment of the present invention is extremely useful for developing and selecting suitable materials in a high strength steel material in which it is necessary to counter hydrogen embrittlement. Below, the method for evaluation of hydrogen embrittlement according to an embodiment of the present invention will be explained in more detail.

[Stress Applying Step]

In the method for evaluation of hydrogen embrittlement according to an embodiment of the present invention, stress is applied to a test piece of a steel material to give a value of stress triaxiality selected in advance from a range of 0.30 to 0.80. As explained above, in the stress state of uniaxial tension, the stress triaxiality becomes 0.33. As opposed to this, the value becomes higher the more the multiaxiality increases. However, in studies by the inventors, a stress state where the stress triaxiality becomes more than 0.80 has not been confirmed in parts of automobiles, etc., both at the time of production and at the time of use. Therefore, by selecting a suitable value of stress triaxiality from a range of 0.30 to 0.80 in accordance with the part at which the steel material is used and its portion, it is believed possible to suitably simulate the stress state in an actual part. The lower limit of the stress triaxiality may, for example, be 0.33 or more, 0.36 or more, 0.40 or more, or 0.45 or more. Similarly, the upper limit of the stress triaxiality may, for example, be 0.75 or less, 0.70 or less, 0.66 or less, or 0.60 or less.

As the technique for selecting stress triaxiality in advance, any suitable method known to a person skilled in the art is possible. The method is not particularly limited. For example, CAE (computer aided engineering) analysis may be mentioned. By using CAE analysis or another analysis technique, it is possible to repeatedly design and evaluate parts to which a steel material will be applied, etc., without fabricating prototypes. Furthermore, by using CAE analysis or another analysis technique, it becomes possible to suitably calculate the stress triaxiality at specific portions of parts having specific shapes to be evaluated for hydrogen embrittlement, for example, the top parts of bends and the surface parts of steel materials at which hydrogen embrittlement cracking can easily occur. In addition, it is possible to relatively easily find the stress required to be applied to a test piece of a steel material for obtaining a preselected value of stress triaxiality. The CAE analysis can be performed by using any suitable software (for example, Marc made by MSC Software, etc.) to, for example, create an analysis model by the finite element method (FEM). At the time of preparation of the analysis model, the mesh size and other necessary conditions may be suitably determined considering the precision of analysis and time required for analysis. As another technique for selecting the stress triaxiality in advance, for example, the method using X-ray diffraction may be mentioned. More specifically, it is also possible to use a prototype of a part to which the steel material is to be applied, etc., to measure the stress by X-ray diffraction and use the obtained data as the basis to find in advance the relationship between the value of the stress triaxiality at a specific portion of the part and the stress necessary to be applied for obtaining that.

[Applying Stress by Bending, Etc.]

The stress to be applied to a test piece of a steel material, for example, the stress to be found by CAE analysis, etc., based on a preselected stress triaxiality, can be given by any suitable operation. While not particularly limited, for example, stress can be applied by bending, stretching, tensioning, extruding, torsion, etc. Preferably, the stress is applied by bending and stretching. Most of the stress applied when assembling an automobile is bending stress. Further, bending is an operation very easily susceptible to the effects of the steel material surface. For example, if the steel material surface is covered by an Al plating, etc., the surface becomes hard, therefore hydrogen embrittlement cracking sometimes easily occurs. As a conventional method for evaluation of hydrogen embrittlement, other than one using the previously explained U-bent test piece, for example, there is one using a cut tensile test piece. However, evaluation of hydrogen embrittlement using a cut tensile test piece is not affected much at all by the steel material surface compared with the case of bending. For this reason, in a conventional method using such a test piece, if considering an actual part, sometimes suitable evaluation is not necessarily performed. Therefore, by applying stress in the method for evaluation of hydrogen embrittlement by bending, evaluation in an environment closer to an actual part becomes possible. However, even if simply applying stress by bending, if the stress state differs as explained above, sometimes suitable evaluation is not necessarily possible. Therefore, it becomes important to apply suitable bending stress to a test piece of a steel material to give the value of stress triaxiality preselected from a range of 0.30 to 0.80.

A further advantage of applying stress by bending is that it is possible to change a ratio w/t of a width "w" (mm) and thickness "t" (mm) of a test piece so as to relatively easily control the value of the stress triaxiality. Such an advantage was found by the inventors this time. If explaining this in more detail, for example, if a width "w" of a test piece is relatively small compared with a thickness "t", the test piece becomes an elongated shape and, even if bent, the stress state becomes substantially one close to uniaxial tension. For this reason, if making the value of the w/t of the test piece smaller, the stress triaxiality approaches 0.30, in particular 0.33. On the other hand, if the width "w" is sufficiently larger than the thickness "t", displacement of the test piece with respect to stress is constrained and the stress state becomes one of plane strain tension with a higher multiaxiality. For this reason, by increasing the value of the w/t of the test piece, it becomes possible to realize a stress state having a desired stress triaxiality higher than 0.30, particularly higher than 0.33.

The w/t of a test piece may be suitably selected in value in accordance with a preselected stress triaxiality. While not particularly limited, for example, it is preferably selected from a range of 1.00 to 30.00. From the viewpoint of controlling the stress triaxiality to a higher value, the w/t may also, for example, be 2.00 or more, 3.00 or more, 5.00 or more, 10.00 or more, or 15.00 or more. In the case of applying stress by bending, the stress state of a test piece is affected by the curvature, distance between support points of the test piece, and other conditions aside from the w/t of a test piece. For this reason, the specific value of w/t has to be suitably selected in each evaluation method. However, even if making w/t excessively large from the viewpoint of control of the stress triaxiality to the desired value, the effect becomes saturated and control to a stress triaxiality higher than a certain value becomes difficult. For this reason, the w/t is preferably 30.00 or less, for example, is 28.00 or less, 25.00 or less, 22.00 or less, or 20.00 or less. The w/t of the test piece can be easily controlled by, for example, maintaining the thickness "t" constant while changing the width "w".

As specific examples of bending, for example, 4-point bending, 3-point bending, U-bending, etc., are included. The suitable bending method can be selected in accordance with the desired stress triaxiality. More specifically, if stress is applied by 4-point bending, it is possible to make the w/t of the test piece change, for example, make the w/t of the test piece change within a range of 1 to 30, to control the stress triaxiality to a range of 0.30 to 0.50, in particular, to 0.33 to 0.45.

[Applying of Stress by Spherical Punching, Etc.]

On the other hand, if the value of the preselected stress triaxiality is higher, sometimes it is not necessarily possible to realize the desired stress triaxiality with applying stress by bending. As opposed to this, by utilizing equibiaxial tension, it is possible to realize 0.66 stress triaxiality. However, to perform equibiaxial tension, the test apparatus becomes relatively large in size and sometimes will not necessarily be suitable. Therefore, the inventors studied a method of applying stress realizing higher stress triaxiality relatively easily and reliably. As a result, the inventors discovered that by utilizing spherical punching, it is possible to realize a high stress triaxiality equal to the equibiaxial tension. Further, spherical punching has the advantages of being more readily affected by the steel material surface in the same way as the above-mentioned bending and enabling evaluation close to an actual car body part.

Spherical punching can be used to apply stress by, for example, spherical punching fixing a test piece to a die having a predetermined hole diameter, then generally pressing a spherical head punch having a predetermined diameter smaller than the hole diameter against the test piece. Therefore, it is possible to apply stress by spherical punching relatively compactly and easily. In addition, the top part of the spherical head becomes a stress state close to equibiaxial tension, therefore higher stress triaxiality difficult to reliably realize with bending, specifically 0.55 to 0.65 or 0.57 to 0.65 stress triaxiality, can be realized. Further, by suitably changing the shape of a test piece, it is also possible to further change the stress state of the test piece. For example, by changing the shape of a test piece from a square shape to that square shape with one pair of facing parallel parts squeezed in, it is possible to change the value of the stress triaxiality to a lower value, for example, to 0.45 to 0.55 or 0.45 to 0.59. In an automobile part, for example, a frame member such as a pillar member, such a relatively high stress state can arise. For this reason, applying stress by spherical punching enables creation of stress states which were not possible to simulate by conventional methods for evaluation of hydrogen embrittlement and is extremely useful for the development and selection of suitable materials in high strength steel materials requiring hydrogen embrittlement to be dealt with.

As explained above, by applying stress by bending and applying stress by spherical punching, it is possible to comprehensively cover at least stress states corresponding to uniaxial tension to stress states corresponding to equibiaxial tension. Therefore, according to an embodiment of the present invention, it is possible to suitably selectively use these methods of applying stress in accordance with the part to be evaluated for hydrogen embrittlement and furthermore suitably change the shape of individual test pieces so as to enable more accurate evaluation of hydrogen embrittlement in an environment closer to an actual part. Further, by applying triaxial tension, it is possible to create a further higher stress state than a stress state corresponding to equibiaxial tension, more specifically possible to create a stress state corresponding to a 0.65 to 0.80 stress triaxiality. Therefore, the method for evaluation of hydrogen embrittlement according to an embodiment of the present invention can be usefully applied in the evaluation of steel materials in the automotive field of course, but also in other technical fields where both high strength and excellent hydrogen embrittlement resistance are sought.

[Step of Determining Critical Hydrogen Content]

In the method for evaluation of hydrogen embrittlement according to an embodiment of the present invention, the test piece to which stress is applied in the stress applying step is determined for the critical hydrogen content (Hc) of the steel material at the next step. In the present invention, the "critical hydrogen content (Hc)" means the upper limit content of hydrogen where the steel material does not crack by hydrogen embrittlement. The way the critical hydrogen content of the steel material is determined is not particularly limited. The content may be determined by any suitable method known to persons skilled in the art.

For example, first, a test piece cut out in a predetermined shape so as to match with the specific test method, and hydrogen is introduced into the test piece by any suitable method. Then, stress is applied to the test piece in the previously explained stress applying step. If evaluating the hydrogen embrittlement of a steel material at the time of production of automobiles, etc., in the next step of determining the critical hydrogen content, the test piece to which stress has been applied in this manner is for example allowed to stand indoors for one to five days. By allowing it to stand indoors for one to five days, hydrogen is sufficiently made to diffuse into the test piece, then the test piece is examined for the presence of any cracking. At the same time as this, temperature programmed desorption is used to heat the test piece, then the amount of introduction of hydrogen is measured from the amount of hydrogen released at that time. The temperature programmed desorption can be performed by heating the test piece from room temperature to 250°C or 300°C by a predetermined temperature raising speed, for example, a 100°C/h temperature raising speed. The test piece for measurement of the amount of introduction of hydrogen may be a separate test piece from the test piece to which stress has been applied, i.e., stress need not be applied if hydrogen was introduced by a method the same as a test piece to which stress has been applied. However, sometimes stress causes the amount of introduction of hydrogen to change, therefore if comparing steel materials with each other, it is better to measure the amount of hydrogen at a test piece not given stress. On the other hand, if evaluating the hydrogen embrittlement of a steel material at the time of use of an automobile, etc., it is possible to allow a test piece given stress to stand in an outdoor exposure test for up to a maximum of 10 years in accordance with the envisioned corrosive environment or to perform a cyclic corrosion test (CCT) or other accelerated test under suitable conditions, then similarly observe the test piece for any cracking and determine the greatest amount of introduction of hydrogen where no cracking occurs as the critical hydrogen content.

The timing of introduction of hydrogen is not necessarily limited, but for example hydrogen can be introduced before applying stress to a test piece cut out from the steel material. Further, hydrogen may be introduced to the steel material before cutting out the test piece. However, in this case, the test piece is preferably cooled by dry ice, etc., so that the hydrogen is not desorbed when working the test piece. Alternatively, further, from the viewpoint of simulating a painting step, for example, hydrogen may also be introduced in the state where stress has been applied to the test piece after applying stress by 4-point bending, etc. However, in this case, preferably hydrogen is not introduced by the conventionally known Cd plating or the later explained galvanization. If simulating introduction of hydrogen at the time of high temperature heating in hot stamping, preferably hydrogen is introduced to the steel material before cutting out the test piece. On the other hand, if simulating hydrogen embrittlement cracking in a process of production of an automobile, etc., hydrogen may be introduced to a test piece before being given stress cut out from a steel material or a test piece after being given stress by bending, etc.

The method for introduction of hydrogen as well is not particularly limited. It is possible to employ any suitable means known to persons skilled in the art. For example, hydrogen can be introduced by dipping the steel material or test piece in a solution containing hydrochloric acid or thiocyanic acid or other hydrogen supply source or by chemical conversion or electrodeposition of a steel material or test piece. In the case of a hot stamping material, for example, hydrogen can be introduced by dew point control during heat treatment in addition to the above methods of introduction. More specifically, the steel material may be heated to a predetermined temperature, for example, about 900°C or a temperature higher than that in a heating furnace with a dew point controlled to a predetermined value within a range of -30 to +50°C for example for 120 to 1000 seconds to thereby introduce hydrogen from the moisture in the atmosphere. By doing this, it is possible to simulate the introduction of hydrogen at the time of high temperature heating in hot stamping. Further, it is possible to change the dew point of the heating furnace and/or holding time of the steel material so as to control the amount of introduction of hydrogen into the steel material. More specifically, it is possible to raise the dew point and/or lengthen the holding time so as to increase the amount of hydrogen introduced into the steel material. Similarly, it is possible to lower the dew point and/or shorten the holding time so as to reduce the amount of hydrogen introduced into the steel material.

On the other hand, in the case of high strength cold rolled steel sheet, if performing heat treatment at a high temperature such as explained in relation to a hot stamping material, the metallostructure changes and sometimes the strength ends up falling. Therefore, from the viewpoint of simulating hydrogen embrittlement cracking when cold working high strength cold rolled steel sheet or when welding to assemble parts, for example, it is preferable to dip the steel material or test piece in a solution containing hydrochloric acid or thiocyanic acid or other hydrogen supply source so as to introduce hydrogen. In this case, the test piece, etc., can be dipped in the solution containing the hydrogen supply source: For example, a test piece given stress by 4-point bending, U-bending, etc., using a predetermined fixture, can be dipped together with the fixture in a solution containing hydrochloric acid or thiocyanic acid or other hydrogen supply source and having a predetermined pH or concentration for example for 24 to 72 hours. In the case of dipping in hydrochloric acid, for example, it is possible to change the pH of the hydrochloric acid aqueous solution to control the amount of introduction of hydrogen, more specifically it is possible to reduce the pH to make the solution more acidic so as to increase the amount of introduction of hydrogen. Similarly, in the case of dipping in thiocyanic acid, it is possible to change concentration of the solution containing thiocyanic acid, for example, an ammonium thiocyanate aqueous solution, so as to control the amount of introduction of hydrogen, more specifically possible to increase the concentration of the ammonium thiocyanate aqueous solution so as to increase the amount of introduction of hydrogen.

The method for cutting out (working) the test piece is not particularly limited. Any suitable method, for example, cutting by electrodischarge wire or laser, etc., can be used. However, an end face after cutting by electrodischarge wire or laser, etc., is preferably finished by polishing by a grindstone, etc., so as to remove any heat affected zone formed by the cutting. Further, for example, if introducing hydrogen before working a test piece, there is the problem that hydrogen will end up dissipating from the cut end face after working the test piece. On the other hand, if introducing hydrogen after working the test piece, after introduction of hydrogen, similarly there is the problem that hydrogen will end up dissipating from the cut end face of the test piece. If such dissipation of hydrogen occurs, sometimes it is not possible to more accurately simulate the state of penetration of hydrogen in an actual part. Explained more specifically, a steel material surface is generally plated, but a cut end face after working is not plated. For this reason, the hydrogen penetrating steel during production can mainly dissipate from the cut end face which is not plated. However, in an actual part in an automobile, etc., when comparing the area of the part itself and the area of the cut end face, the area of the part itself is far larger, therefore the amount of hydrogen penetrating the part is not necessarily greatly affected by dissipation of hydrogen from the cut end face. In contrast to this, a test piece used in the method for evaluation of hydrogen embrittlement is far smaller in overall size if compared with an actual part. Despite this, for example, in the case of a short strip shaped test piece, since a cut end face having the same thickness (sheet thickness) as an actual part surrounds the four sides, the ratio of the area of the cut end face to the area of the test piece as a whole becomes extremely large. For this reason, the amount of hydrogen introduced to the test piece is more greatly affected relatively by dissipation of hydrogen when compared with an actual part. Therefore, to more accurately simulate the state of penetration of hydrogen at an actual part, it is preferable to suppress or prevent dissipation of hydrogen from the cut end face of the test piece.

[Sealing in Introduced Hydrogen]

In relation to this, in a preferred embodiment of the present invention, the test piece into which hydrogen was introduced is galvanized. By galvanizing at least the cut end face of the test piece into which hydrogen was introduced, preferably the entire surface of the test piece, it becomes possible to reliably suppress or prevent the dissipation of the introduced hydrogen from the cut end face, etc. The galvanization is preferably electrogalvanization. In a preferred embodiment of the present invention, the galvanization is performed for the purpose of sealing in hydrogen rather than corrosion resistance. Therefore, it is enough that the test piece as a whole be uniformly galvanized at a thickness sufficient for sealing in the hydrogen introduced into the test piece. From such a viewpoint, as the galvanization, electrogalvanization is rather preferable to hot dip galvanization. The thickness of the galvanization is, for example, 5 $\mu$m or more, more preferably 10 $\mu$m or more or 15 $\mu$m or more, most preferably 20 $\mu$m or more. In relation to this, in experiments by the inventors, it was confirmed that if galvanizing the entire surface of the test piece to a 20 $\mu$m or more thickness, it is possible to completely or substantially completely prevent the dissipation of hydrogen from the cut end face, etc., of the test piece. In this case, if considering the fact of the cut end face at an actual part is not galvanized, etc., in an actual part, as explained above, the area of the part as a whole is far larger than the area of the cut end face, but hydrogen can dissipate from the cut end face. In contrast to this, according to a preferred embodiment of the present invention, if galvanizing the test piece to a 20 $\mu$m or more thickness, it is possible to conduct the evaluation in the state with the initially introduced hydrogen completely or substantially completely sealed inside the test piece and becomes possible to more toughly evaluate hydrogen embrittlement compared with the case of an actual part, at least equally evaluate it.

To make the above effect when performing galvanization more prominent, the test piece into which hydrogen was introduced is preferably galvanized as fast as possible before the dissipation of hydrogen from the cut end face, etc. For example, if hydrogen is introduced after working the test piece, the test piece is galvanized within 30 minutes after introduction of hydrogen, preferably within 10 minutes. Similarly, if introducing hydrogen to the steel material, then working the test piece, the test piece is galvanized within 30 minutes after working the test piece, preferably within 10 minutes.

In addition to or in place of galvanization, after introduction of hydrogen to the test piece or during the subsequent operation for working the test piece, the operation for sealing in the hydrogen, the operation for applying stress, and other operations, the test piece is preferably stored cooled by dry ice (more specifically, solid state dry ice), an organic solvent cooled by dry ice, or dipping in liquid nitrogen (refrigerated handling). By storing the test piece cooled by dry ice, it is possible to suppress or reduce the dissipation of hydrogen from the cut end face, etc., of the test piece. For this reason, it is possible to more accurately simulate the state of penetration of hydrogen in an actual part.

[Steel Material]

The steel material to which the method for evaluation of hydrogen embrittlement according to an embodiment of the present invention is applied may be any steel material in which hydrogen embrittlement can occur. It is not particularly limited, but for example the steel material may be a so-called high strength cold rolled steel sheet, more specifically a high strength cold rolled steel sheet having a 980 MPa or more tensile strength, and may be a steel material for hot stamping use or a hot stamped body shaped by hot stamping. In general, along with higher strength of a steel material, evaluation of hydrogen embrittlement in an environment closer to an actual part such as of an automobile becomes difficult. However, in the method for evaluation of hydrogen embrittlement according to an embodiment of the present invention, it is possible to realize more suitable evaluation of hydrogen embrittlement even in high strength steel sheet by considering the stress state of a test material and applying to a test piece of the steel material a suitable stress utilizing stress triaxiality. Therefore, the method for evaluation of hydrogen embrittlement according to an embodiment of the present invention can be applied to a steel material having an extremely high strength, for example, can be applied to a steel material having a 980 MPa or more, 1180 MPa or more, 1470 MPa or more, 1600 MPa or more, 1800 MPa or more, or 2000 MPa or more tensile strength. The upper limit of the tensile strength is not particularly prescribed, but, for example, the tensile strength of the steel material may be 3150 MPa or less, 2850 MPa or less, or 2500 MPa or less. The tensile strength of the steel material is measured by fabricating a No. 5 test piece and conducting a tensile test based on JIS Z 2241:2022. If there are limitations on the size of the steel material and a JIS No. 5 test piece cannot be obtained, it is also possible to use a ASTM1/2 test piece based on ASTM Standard E8.

[Covering]

The steel material may be provided with a covering at part or all of the surface. The "covering" means a coating, plating layer, alloyed plating layer, and intermetallic compound layer. For example, by applying a covering similar to the covering applied to an actual part to a test piece, it becomes possible to more accurately evaluate the hydrogen embrittlement in an environment closer to the actual part. The covering may be a covering mainly comprised of an Fe-Al-based alloy and may be a covering mainly comprised of an Fe-Zn-based alloy. The "covering mainly comprised of an Fe-Al-based alloy" is a covering containing Fe and Al in a total of 70 mass% or more, while the "covering mainly comprised of an Fe-Zn-based alloy" is a covering containing Fe and Zn in a total of 70 mass% or more. Further, the covering may also be an Al plating layer, Al-Zn plating layer, Al-Si plating layer, hot dip galvanized layer, electrogalvanized layer, hot dip galvannealed layer, Zn-Ni plating layer, and Al-Mg-Zn-based plating layer. If the covering contains Al, the Fe-Al-based alloy formed at the interface of the covering and steel material functions as a barrier to hydrogen, therefore it is possible to reduce or suppress the dissipation of hydrogen from the steel material to the outside. Therefore, from the viewpoint of more accurate evaluation of hydrogen embrittlement, the steel material may also be provided with a covering containing Al. The thickness of the covering is not particularly limited, but in general is preferably 10 to 100 $\mu$m.

[Step of Calculating Stress Conditions]

The method for evaluation of hydrogen embrittlement according to a preferred embodiment of the present invention further comprises calculating stress triaxiality and maximum main stress at a specific portion in a part, in particular a steel part to be evaluated for hydrogen embrittlement before applying stress to the test piece of the steel material,
wherein applying stress to the test piece of the steel material comprises applying a stress corresponding to the calculated stress triaxiality and maximum main stress to the test piece of the steel material.

As explained previously, stress triaxiality, as shown in the following formula 1 is a parameter of the average stress (of three axes) divided by the equivalent stress. The average stress and equivalent stress are respectively expressed by the following formulas 2 and 3 using the three components $\sigma_1$, $\sigma_2$, and $\sigma_3$ of main stress:

$$\text{Stress triaxiality} = \text{average stress/equivalent stress} \qquad \cdots \quad \text{formula 1}$$

[Mathematical 3]

$$\text{Average stress} = \frac{1}{3}(\sigma_1 + \sigma_2 + \sigma_3) \qquad \cdots \quad \text{formula 2}$$

[Mathematical 4]

$$\text{Equivalent stress} = \sqrt{\frac{1}{2}\{(\sigma_1 - \sigma_2)^2 + (\sigma_2 - \sigma_3)^2 + (\sigma_3 - \sigma_1)^2\}} \qquad \cdots \quad \text{formula 3}$$

Here, the "maximum main stress" means the largest of the three components $\sigma_1$, $\sigma_2$, and $\sigma_3$ of the main stress. Alternatively, further, it is possible to define the maximum main stress ($\sigma_1$), intermediate main stress ($\sigma_2$), and minimum main stress ($\sigma_3$) in the order of magnitude of value, i.e., define $\sigma_1$ as the maximum main stress. In a preferred embodiment of the present invention, by utilizing CAE analysis or another analytical technique, the stress triaxiality and maximum main stress at the part to be evaluated for hydrogen embrittlement, in particular a specific portion in a steel part, for example, a top part of a bend or surface part of a steel material where hydrogen embrittlement cracking easily occurs are calculated and stress corresponding to the calculated stress triaxiality and maximum main stress is applied to the test piece of the steel material. At this time, the inventors discovered that when applying stress in the stress applying step, in particular if applying a stress corresponding to the stress triaxiality and maximum main stress to a test piece, it is possible to more stably determine the critical hydrogen content of the steel material without being greatly affected by the stress state of the steel material.

If explained more specifically, the critical hydrogen content, as explained previously, means the upper limit of the amount of hydrogen at which hydrogen embrittlement cracking will not occur at the steel material. In general, the higher the strength of the steel material, the smaller that value will become. Therefore, even if making a steel material high in strength, if possible to maintain the critical hydrogen content of the steel material at a certain high value or more, the steel material can be evaluated as being excellent in hydrogen embrittlement resistance. On the other hand, if the conditions for the stress applied are not constant, variation occurs in the critical hydrogen content obtained, therefore it is not possible to correctly evaluate the hydrogen embrittlement resistance of the steel material. For example, in the case of testing the same steel material, if the stress applied is high in one test and the stress is low in another test, in the test at which the high stress is applied, the critical hydrogen content of the steel material calculated ends up becoming smaller. For this reason, in the conventional method for evaluation of hydrogen embrittlement, a strain gauge, etc., is utilized and a U-bending test, etc., is performed to apply a predetermined stress to a test piece to thereby keep the critical hydrogen content of the steel material calculated from fluctuating due to the stress applied. However, as explained previously, in such an evaluation method as well, the stress states will sometimes differ between a U-bending test or other test and a separately preformed tensile test using uniaxial tension and furthermore with the actual part. In such a case, sometimes suitable stress cannot necessarily be applied in a test for evaluation of hydrogen embrittlement. As opposed to this, according to an embodiment of the present invention, instead of a conventional method of evaluation using only a strain gauge, it is possible to simulate various stress states in actual parts by applying suitable stress to a steel material considering stress triaxiality. As a result, the upper limit of the amount of hydrogen at which hydrogen embrittlement cracking does not occur in a steel material, i.e., the critical hydrogen content, can be more accurately calculated compared with a conventional evaluation method.

According to a preferred embodiment of the present invention, by applying stress corresponding to the stress triaxiality and the highest main stress among the main stresses $\sigma_1$, $\sigma_2$, and $\sigma_3$ (i.e., the maximum main stress) to a test piece of a steel material, for example, it becomes possible to more stably determine the critical hydrogen content without the critical hydrogen content of the steel material greatly fluctuating due to the value of the stress triaxiality. While not intending to be bound by any specific theory, in actual parts, while there are various stress states, it is believed that the hydrogen embrittlement property of a steel material is determined by the highest stress among the load stresses of the various directions forming that stress state. Therefore, in the method for evaluation of hydrogen embrittlement as well, rather than the absolute value of the stress applied being important, it can be said to be important to more concretely obtain a grasp of the stress state at a specific portion of a part to be evaluated for hydrogen embrittlement by utilizing stress triaxiality. In the step for calculating the stress conditions, it is possible to calculate the stress triaxiality and the maximum main stress forming such a stress state and find the stress corresponding to the calculated stress triaxiality and maximum main stress, more specifically the stress required for reproducing the calculated stress triaxiality and maximum main stress at a test piece in a test for evaluation of the hydrogen embrittlement (or amount of displacement of test piece). If applying the thus found stress to the test piece in the next stress applying step, it is believed that a suitable stress will be applied in an environment closer to the actual part, therefore it becomes possible to more accurately evaluate hydrogen embrittlement.

The method for obtaining a grasp of the stress state in an actual part, i.e., the calculation of the stress triaxiality and maximum main stress and the calculation of the stresses, etc., required for reproducing the stress triaxiality and maximum main stress in a test piece, is not particularly limited, but this can be relatively easily performed by utilizing CAE analysis or other analytical technique.

<Device for Evaluation of Hydrogen Embrittlement>

Next, a device for evaluation of hydrogen embrittlement suitable for performing the method for evaluation of hydrogen embrittlement explained above will be explained. However, the evaluation device is not limited to use in such a specific evaluation method and needless to say can be applied in any evaluation method for evaluating the hydrogen embrittlement resistance of a steel material.

The device for evaluation of hydrogen embrittlement according to an embodiment of the present invention comprises

a holder for holding a test piece of a steel material,
a stress applying means for applying stress to the test piece,
a display part for displaying an amount of displacement of the test piece to which stress has been applied, and
a transmitting means for mechanically transmitting the amount of displacement of the test piece to the display part,
wherein the device is able to control the stress applied by the stress applying means so that the amount of displacement of the test piece becomes an amount of displacement corresponding to a predetermined value of stress triaxiality.

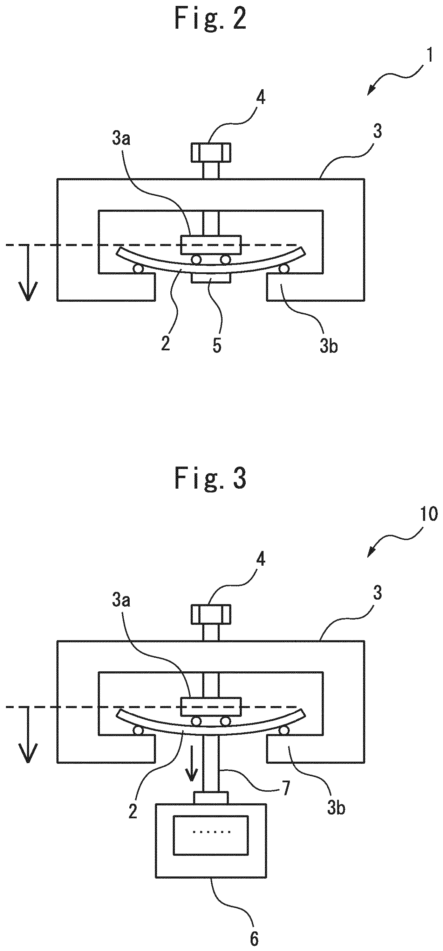

FIG. 2 is a schematic view showing a conventional device for evaluation of hydrogen embrittlement. Referring to FIG. 2, the conventional device 1 for evaluation of hydrogen embrittlement is comprised of a holder 3 for holding a test piece 2 of a steel material, a stress applying means 4 for applying stress to the test piece 2, and a strain gauge 5 attached to a bent top of the test piece 2. The strain gauge 5 is generally structured as a thin insulator on which a metal resistor is laid out in a zigzag pattern and is able to measure the change in electrical resistance accompanying deformation of the resistor and calculate the amount of strain of a measured object (test piece). Further, in FIG. 2, as the holder 3, a structure comprised of a load fixture 3a and a receiving fixture 3b is shown. It is designed to enable performance of a 4-point bending test bending a test piece by the two support points of the load fixture 3a and the two support points of the receiving fixture 3b. In the conventional device 1 for evaluation of hydrogen embrittlement, as explained in relation to FIG. 1(d), the stress applied to the test piece 2 is determined utilizing a stress-strain curve obtained by a tensile test, etc., performed separately using the same strain gauge. However, in this case, even if the value of stress based on the amount of strain is the same between the evaluation test and a separately performed tensile test, etc., sometimes the stress states of the test pieces in the respective tests differ. Further, sometimes similarly the stress states differ between the test piece used at the evaluation test and the actual part. In such a case, only naturally, sometimes it is not possible to suitably evaluate a steel material for hydrogen embrittlement.

FIG. 3 is a schematic view showing a device for evaluation of hydrogen embrittlement according to an embodiment of the present invention. Referring to FIG. 3, the device 10 for evaluation of hydrogen embrittlement according to an embodiment of the present invention is comprised of a holder 3 for holding a test piece 2 of a steel material, a stress applying means 4 for applying stress to the test piece 2, a display part 6 for displaying an amount of displacement of the test piece 2 to which stress has been applied, and a transmitting means 7 for mechanically transmitting the amount of displacement of the test piece 2 to the display part 6. In FIG. 3 as well, as the holder 3, a structure comprised of a load fixture 3a and a receiving fixture 3b is shown and it is made possible to perform a 4-point bending test for bending the test piece by the two support points of the load fixture 3a and the two support points of the receiving fixture 3b. In the device 10 for evaluation of hydrogen embrittlement according to an embodiment of the present invention, if the test piece 2 displaces in the downward direction by such a 4-point bending test, the transmitting means 7 arranged in contact with the displacement part of the test piece 2 pushes the display part 6 downward along the axial direction in accordance with its amount of displacement whereby the amount of displacement is mechanically transmitted to the display part 6 and at the display part 6, the amount of displacement of the test piece 2 is displayed. For this reason, in the device 10 for evaluation of hydrogen embrittlement according to an embodiment of the present invention, unlike the case of a conventional device 1 for evaluation of hydrogen embrittlement, it is not necessarily required to use a strain gauge 5 for electrically detecting the amount of displacement (amount of strain) of the test piece 2. That is, the device 10 for evaluation of hydrogen embrittlement according to an embodiment of the present invention need not be provided with the strain gauge 5. Explained in more detail, in the evaluation device 10, first, CAE analysis or other analysis technique is utilized to calculate in advance the amount of displacement of the test piece 2 corresponding to a predetermined value of the stress triaxiality. Next, stress is applied to the test piece 2 by the stress applying means 4 so that the amount of displacement of the test piece 2 displayed at the display part 6 becomes the amount of displacement calculated in advance. In FIG. 3, a bolt is shown as a stress applying means 4. The bolt passes through the outside wall of the surface holder 3 and contacts the surface of the load fixture 3a in the holder 3 at the opposite side to the surface provided with the two support points. By adopting such a structure, screwing in the bolt used as the stress applying means 4 enables the load fixture 3a to be pushed in and enables 4-point bending of the test piece 2 by the two support points of the load fixture 3a and the two support points of the receiving fixture 3b. According to the evaluation device 10, it is possible to confirm the amount of displacement of the test piece displayed at the display part 6 (in the embodiment shown in FIG. 3, corresponding to the "amount of pushdown") while controlling or adjusting the applying of stress by the stress applying means 4, therefore it becomes possible to realize precision applying of stress. Note that, from the viewpoint of improvement of the accuracy of hydrogen embrittlement, reduction of the load in setting conditions in CAE analysis and other analytical techniques, etc., it is also possible to jointly use a strain gauge so as to confirm whether the amount of displacement calculated by utilizing CAE analysis, etc., is the amount of displacement as targeted. Below, the component elements of the device for evaluation of hydrogen embrittlement according to an embodiment of the present invention will be explained in more detail.

[Test Piece]

The test piece may be one made from any steel material at which hydrogen embrittlement can occur. Specifically, it may be one made from a steel material explained in relation to the method for evaluation of hydrogen embrittlement according to an embodiment of the present invention, for example, a steel material having a 980 MPa or more tensile strength. The shape of the test piece may be any shape suitable for the specific test method. While not particularly limited, for example, in the case of a test by 4-point bending, 3-point bending, U-bending, etc., such as shown in FIG. 3, the test piece may have a short strip shape. Further, in the case of a test by spherical punching, the test piece may have a rectangular or square shape.

[Holder]

The holder for holding a test piece of a steel material is not particularly limited and may have any structure configured to be able to hold a test piece and apply a predetermined stress, for example, stress by 3-point bending, 4-point bending, spherical punching, etc. For example, in the case of applying stress by 4-point bending, as shown in FIG. 3, the holder is comprised of a load fixture and receiving fixture respectively provided with two support points and may hold a test piece between the side of the load fixture where the two support points are provided and the side of the receiving fixture where the two support points are provided. Similarly, for example, in the case of applying stress by 3-point bending, the holder is comprised of a load fixture provided with one support point and a receiving fixture provided with two support points and may hold a test piece between the side of the load fixture where the one support point is provided and the side of the receiving fixture where the two support points are provided.

The holder may be configured to be able to hold a test piece changed in the ratio w/t of width "w" (mm) and thickness "t" (mm) to a range of 1.00 to 30.00. For example, if applying stress using 4-point bending, 3-point bending, or other bending, in general a short strip shaped test piece is used. A wide width test piece is not used. Therefore, a conventional holder used for applying bending stress is not configured so as to be able to hold a test piece changed in the ratio w/t of the width "w" and thickness "t" to a range of 1.00 to 30.00, in particular a wide width test piece. As explained above in relation to the method for evaluation of hydrogen embrittlement, by changing the ratio w/t of the width "w" and thickness "t" of the test piece, it is possible to relatively easily control the value of the stress triaxiality. Therefore, if the holder for holding the test piece is able to hold even a wide width test piece with a relatively large w/t in addition to a conventionally used test piece having a general w/t, by just suitably selecting the shape of the test piece, it becomes possible to simulate various stress states at actual parts in the same device for evaluation of hydrogen embrittlement. The w/t, for example, may be 2.00 or more, 3.00 or more, 5.00 or more, 10.00 or more, or 15.00 or more. Similarly, the w/t may also, for example, be 28.00 or less, 25.00 or less, 22.00 or less, or 20.00 or less. The shape of the holder for holding a test piece able to change the w/t to a range of 1 to 30.00 is not particularly limited, but, for example, the dimensions of the holder in the direction corresponding to the width direction of the test piece, more specifically, the depth direction of the holder shown in FIG. 3, may be made sufficiently larger.

If applying stress by spherical punching, the holder for holding a test piece of a steel material may be provided with a die having a predetermined hole size generally used in, for example, shaping by spherical punching and a spherical head punch compatible with the hole diameter. For example, it is also possible to replace the load fixture 3a of the holder shown in FIG. 3 with a spherical head punch, place in that holder a die having a predetermined hole diameter for spherical punching, and hold a test piece between the same. As explained above in relation to the method for evaluation of hydrogen embrittlement, by applying stress by bending and by applying stress by spherical punching, it is possible to comprehensively cover at least a stress state corresponding to uniaxial tension to a stress state corresponding to equibiaxial tension. For this reason, by applying the holder shown in FIG. 3 for applying stress by spherical punching, it becomes possible to create various stress states by a single device for evaluation of hydrogen embrittlement.

[Stress Applying Means and Transmitting Means]

The stress applying means for applying stress to the test piece is not particularly limited. It may be any suitable means generally used in evaluation of hydrogen embrittlement. For example, as the stress applying means, it is possible to use a bolt or other means able to be screwed in such as illustrated in FIG. 3. A bolt or other means able to be screwed in can reduce the amount of displacement in the axial direction with respect to the amount of rotation during the screw in. For this reason, a bolt or other means able to be screwed in enables precise control of the amount of displacement and is useful for application as a stress applying means. The transmitting means for mechanically transmitting the amount of displacement of the test piece to the display part is not particularly limited and may be any structure able to mechanically transmit the amount of displacement of the test piece by the stress applying means to the display part. For example, if applying stress by 4-point bending, 3-point bending, spherical punching, etc., the amount of displacement of the test piece can handle the amount of pushdown such as shown in FIG. 3. Therefore, in the case of such an embodiment, the transmitting means is preferably a circular column, polygonal column, or other columnar member having an axial direction length. By placing such a columnar member in contact with a displacement part of the test piece as a transmitting means, as shown in FIG. 3, the transmitting means pushes the display part downward along the axial direction in accordance with the amount of displacement of the test piece, therefore it is possible to mechanically reliably transmit the amount of displacement to the display part.

[Display Part]

The display part for displaying the amount of displacement of the test piece to which stress has been applied is not particularly limited. It may be any device which can convert the amount of displacement mechanically transmitted by a transmitting means to an electrical signal, etc., for display. According to a device for evaluation of hydrogen embrittlement according to an embodiment of the present invention, for example, it is possible to calculate in advance the amount of displacement of a test piece corresponding to a predetermined stress triaxiality by CAE analysis, etc., and confirm the amount of displacement of a test piece displayed at the display part while controlling the applying of stress by the stress applying means so that the amount of displacement becomes the amount of displacement calculated in advance and therefore possible to realize precision applying of stress.

While not shown in FIG. 3, a device for evaluation of hydrogen embrittlement according to an embodiment of the present invention may further comprise a stand for setting the evaluation device. The stand may be any structure able to fix the positional relationship between the holder and display part in the device for evaluation of hydrogen embrittlement. By fixing the positional relationship of the holder and the display part, even if stress is applied to a test piece due to a pushdown operation by the stress applying means, etc., the holder and the display part will not move and their positional relationship will be maintained. For this reason, it becomes possible to reliably realize precision applying of stress. The method of fixing the relationship is not particularly limited. For example, the stand may be provided with a setting part for setting the holder and/or the display part, the holder and/or the display part may be fixed to the setting part, or the holder and/or the display part may be fixed to the side wall part of the stand.

Below, examples will be used to explain the present invention in more detail, but the present invention is not limited to these examples in any way.

EXAMPLES

In the following examples, the method for evaluation and evaluation device of hydrogen embrittlement according to an embodiment of the present invention were used to evaluate the hydrogen embrittlement of the steel material in various stress states and calculate the critical hydrogen content (Hc) in each evaluation.

[B1 to B10]

First, a steel material of each of the chemical compositions shown in Table 1 was loaded into a heating furnace with a dew point controlled to a predetermined value within a range of -30 to +50°C and heated to 920°C, then was held there for a predetermined time within a range of 120 to 1000 seconds to introduce hydrogen into the steel material from the moisture in the atmosphere. Next, the steel material was taken out from the heating furnace and sandwiched between plate shaped dies at a temperature of room temperature or so to be rapidly cooled and thereby obtain a hot stamped material. From the hot stamped material into which hydrogen was introduced, a short strip shaped test piece for 4-point bending having a w/t (ratio of width "w" and thickness "t" of test piece) or square shaped test piece for spherical punching shown in B1 to B10 of Table 2 was fabricated. During the working, the test piece was handled kept cooled by dry ice. Next, the test piece was electrogalvanized to seal in the hydrogen introduced into the test piece. The test piece with hydrogen sealed in was placed in the holder of the device for evaluation of hydrogen embrittlement shown in FIG. 3, stress was applied to the test piece by 4-point bending or spherical punching to give the values of stress triaxiality and maximum main stress shown in Table 2, then the test piece was held for the maximum for 72 hours to check for the presence of any cracking. The values of the stress triaxiality and maximum main stress were calculated in advance using CAE analysis.

More specifically, when calculating the value of the stress triaxiality, a tensile test of the stee material was performed and the obtained strain-stress curve was used for CAE analysis. The tensile test was performed based on ISO Standard 6892-1:2009. CAE analysis was performed using Marc made by MSC Software and modeling of 4-point bending and spherical punching by the finite element method. At the time of modeling by the finite element method, the mesh size was made 0.2 mm and the friction coefficient $\mu$ of a contact part of the test piece and evaluation device was made 0.05.

The conditions of the heating furnace were changed to make the amount of introduction of hydrogen change and the test was repeated. After the test, the test piece was examined for the presence of any cracks. The greatest amount of introduction of hydrogen where no cracks occurred was determined as the critical hydrogen content (Hc). The amount of introduction of hydrogen was determined by heating the test piece into which hydrogen was introduced under the same conditions by temperature programmed desorption and measuring the amount from the amount of hydrogen released at that time. The temperature programmed desorption was performed by heating the test piece from room temperature to 250°C by a 100°C/h temperature raising speed. The obtained results are shown in Table 2.

[B11 and B12]

In these examples, hydrogen embrittlement of the steel materials was evaluated in the case of fabricating test pieces, applying stress, then introducing hydrogen from the viewpoint of simulating a painting step. First, a steel material of each of the chemical compositions shown in Table 1, unlike the case of B1 to B10, was heated and held under conditions not introducing hydrogen. More specifically, the above steel material was loaded into a heating furnace with a dew point controlled to a predetermined value of -30 and heated to 920°C, then was held there for 120 seconds. Next, the steel material was taken out from the heating furnace and sandwiched between plate shaped dies at a temperature of room temperature or so to be rapidly cooled and thereby obtain a hot stamped material. From the hot stamped material, a short strip shaped test piece for 4-point bending having a w/t (ratio of width "w" and thickness "t" of test piece) or square shaped test piece for spherical punching shown in B11 and B12 of Table 2 was fabricated. Next, the fabricated test piece was placed in the holder of the device for evaluation of hydrogen embrittlement shown in FIG. 3, stress was applied to the test piece by 4-point bending or spherical punching to give the values of stress triaxiality and maximum main stress shown in Table 2. The test piece applied with stress was dipped along with the fixture at a 0.1 to 10% predetermined concentration ammonium thiocyanate aqueous solution, then held for 72 hours to check for the presence of any cracking. In the same way as the case of B1 to B10, the values of the stress triaxiality and maximum main stress were calculated in advance using CAE analysis.

The concentration of the ammonium thiocyanate aqueous solution was changed to make the amount of introduction of hydrogen change and the test was repeated. After the test, the test piece was examined for the presence of any cracks. The greatest amount of introduction of hydrogen where no cracks occurred was determined as the critical hydrogen content (Hc). The amount of introduction of hydrogen was determined by heating the test piece into which hydrogen was introduced under the same conditions by temperature programmed desorption and measuring the amount from the amount of hydrogen released at that time. The temperature programmed desorption was performed by heating the test piece from room temperature to 250°C by a 100°C/h temperature raising speed. The obtained results are shown in Table 2.

[Tensile Strength]

The tensile strength was measured by fabricating a No. 5 test piece from the hot stamped material obtained at B1 to B12 and conducting a tensile test based on JIS Z 2241:2022. The obtained results are shown in Table 2.

[Table 1]

Table 1

| Steel material no. | Chemical composition (mass%), bal.: Fe and impurities | | | | | | | | | | | | | | |
|---|---|---|---|---|---|---|---|---|---|---|---|---|---|---|---|
| | C | Si | Mn | P | S | N | O | Nb | Ti | Cu | Ni | Cr | B | Mo | Al |
| A1 | 0.30 | 0.21 | 1.22 | 0.005 | 0.0005 | 0.003 | 0.004 | 0.05 | 0.037 | | 0.01 | 0.42 | 0.0020 | | 0.03 |
| A2 | 0.34 | 0.39 | 0.66 | 0.008 | 0.0004 | 0.004 | 0.003 | 0.04 | 0.035 | 0.28 | 0.08 | 0.09 | 0.0026 | 0.18 | 0.04 |

[Table 2]

Table 2

| Code | Steel material no. | Tensile strength | Stress triaxiality | Stress applying method | Test piece w/t | Maximum main stress | Critical hydrogen content: Hc |
|---|---|---|---|---|---|---|---|
| | | (MPa) | | | | (MPa) | (mass ppm) |
| B1 | A1 | 1820 | 0.33 | 4-point bending | 3.75 | 1000 | 1.10 |
| B2 | A1 | 1820 | 0.34 | 4-point bending | 6.25 | 1075 | 0.61 |
| B3 | A1 | 1820 | 0.33 | 4-point bending | 3.75 | 1260 | 0.29 |
| B4 | A1 | 1820 | 0.40 | 4-point bending | 12.50 | 1260 | 0.29 |
| B5 | A1 | 1820 | 0.45 | 4-point bending | 18.75 | 1260 | 0.29 |
| B6 | A2 | 2015 | 0.33 | 4-point bending | 3.75 | 1000 | 1.06 |
| B7 | A2 | 2015 | 0.39 | 4-point bending | 12.50 | 1075 | 1.06 |
| B8 | A2 | 2015 | 0.45 | 4-point bending | 18.75 | 1260 | 1.01 |
| B9 | A2 | 2015 | 0.60 | Spherical punching | - | 1260 | 1.01 |
| B10 | A2 | 2015 | 0.60 | Spherical punching | - | 1075 | 1.06 |
| B11 | A1 | 1820 | 0.33 | 4-point bending | 3.75 | 1075 | 0.60 |
| B12 | A1 | 1820 | 0.60 | Spherical punching | - | 1075 | 0.60 |

Referring to Table 2, a trend was seen of the critical hydrogen content (Hc) decreasing along with an increase in the maximum main stress at the tensile strength 1820 MPa and 2015 MPa steel materials. Further, in each of B3 to B5 where stress was applied to the test piece of the tensile strength 1820 MPa Steel Material A1 so that the maximum main stress became 1260 MPa, despite the value of the stress triaxiality being different, the same value of the critical hydrogen content was obtained. The result can be said to show that by the method for evaluation of hydrogen embrittlement according to the present invention, it is possible to accurately calculate the critical hydrogen content of a steel material even while simulating various stress states in actual parts. Further, by changing the w/t of the test piece for 4-point bending, it is possible to control the value of the stress triaxiality, more specifically, by increasing the w/t of the test piece for 4-point bending, it was possible to increase the value of the stress triaxiality. In addition, by utilizing spherical punching instead of 4-point bending, it was possible to further increase the value of stress triaxiality. As a result, it was possible to control the value of the stress triaxiality within a broad range and thereby possible to determine the critical hydrogen content of a steel material in various stress states.

REFERENCE SIGNS LIST

1, 10. device for evaluation of hydrogen embrittlement
2. test piece of steel material
3. holder
3a. load fixture
3b. receiving fixture
4. stress applying means
5. strain gauge
6. display part
7. transmitting means

# EP 4 641 170 A1

**Claims**

1. A method for evaluation of hydrogen embrittlement, comprising applying stress to a test piece of a steel material so as to obtain a value of stress triaxiality selected in advance from a range of 0.30 to 0.80, and determining a critical hydrogen content of the steel material.

2. The method according to claim 1, wherein the stress is applied by bending and the value of the stress triaxiality is controlled by changing a ratio w/t of width "w" and thickness "t" of the test piece.

3. The method according to claim 1, wherein the stress is applied by spherical punching.

4. The method according to any one of claims 1 to 3, wherein determining the critical hydrogen content of the steel material is performed by using a test piece galvanized subsequent to introduction of hydrogen.

5. The method according to any one of claims 1 to 4, further comprising calculating stress triaxiality and maximum main stress at a specific portion in a part to be evaluated for hydrogen embrittlement before applying stress to the test piece of the steel material,
wherein applying stress to the test piece of the steel material comprises applying a stress corresponding to the calculated stress triaxiality and maximum main stress to the test piece of the steel material.

6. A device for evaluation of hydrogen embrittlement, comprising

   a holder for holding a test piece of a steel material,
   a stress applying means for applying stress to the test piece,
   a display part for displaying an amount of displacement of the test piece to which stress has been applied, and
   a transmitting means for mechanically transmitting the amount of displacement of the test piece to the display part,
   wherein the device is able to control the stress applied by the stress applying means so that the amount of displacement of the test piece becomes an amount of displacement corresponding to a predetermined value of stress triaxiality.

7. The device according to claim 6, wherein the predetermined value of stress triaxiality is a value selected from a range of 0.30 to 0.80.

8. The device according to claim 6 or 7, wherein the holder for holding the test piece of the steel material is configured to be able to hold a test piece with a ratio w/t of width "w" and thickness "t" changed within a range of 1.00 to 30.00.

# Fig. 1

(a)  (b)  (c)

STRAIN GAUGE

(d)

STRAIN GAUGE

STRESS

STRAIN

# Fig. 2

# Fig. 3

## INTERNATIONAL SEARCH REPORT

| International application No. |
|---|
| **PCT/JP2023/041468** |

| A. | CLASSIFICATION OF SUBJECT MATTER |
|---|---|

*G01N 17/00*(2006.01)i; *G01N 3/20*(2006.01)i
FI:   G01N17/00; G01N3/20

According to International Patent Classification (IPC) or to both national classification and IPC

| B. | FIELDS SEARCHED |
|---|---|

Minimum documentation searched (classification system followed by classification symbols)

G01N17/00; G01N3/20

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | JP 2011-033600 A (KABUSHIKI KAISHA KOBE SEIKO SHO) 17 February 2011 (2011-02-17) entire text, all drawings | 1-8 |
| A | WO 2019/186898 A1 (NIPPON STEEL CORPORATION) 03 October 2019 (2019-10-03) entire text, all drawings | 1-8 |
| A | KR 10-2022-0146833 A (HYUNDAI STEEL COMPANY) 02 November 2022 (2022-11-02) entire text, all drawings | 1-8 |
| A | JP 2022-130931 A (TORAY INDUSTRIES, INC.) 07 September 2022 (2022-09-07) entire text, all drawings | 1-8 |
| A | JP 2009-069007 A (NIPPON STEEL CORPORATION) 02 April 2009 (2009-04-02) entire text, all drawings | 1-6 |

☐ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **18 December 2023** | **16 January 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2023/041468**

| Patent document cited in search report | | | Publication date (day/month/year) | Patent family member(s) | | | Publication date (day/month/year) |
|---|---|---|---|---|---|---|---|
| JP | 2011-033600 | A | 17 February 2011 | (Family: none) | | | |
| WO | 2019/186898 | A1 | 03 October 2019 | JP | 6573058 | B1 | |
| KR | 10-2022-0146833 | A | 02 November 2022 | (Family: none) | | | |
| JP | 2022-130931 | A | 07 September 2022 | (Family: none) | | | |
| JP | 2009-069007 | A | 02 April 2009 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017142086 A
- JP 2022024814 A
- JP 2008261742 A